# EUROPÄISCHE PATENTSCHRIFT

(11) **EP 2 259 728 B1**
(45) Veröffentlichungstag und Bekanntmachung des Hinweises auf die Patenterteilung: **13.03.2019**
(21) Anmeldenummer: 09725036.9
(22) Anmeldetag: 26.03.2009
(51) Int. Cl.: A61B 17/00, A61F 2/01, A61B 17/12, A61F 2/06, A61F 2/24

(54) **VORRICHTUNG ZUM VERSCHLUSS VON DEFEKTEN IM GEFÄSSSYSTEM**
DEVICE FOR CLOSING DEFECTS IN THE VASCULAR SYSTEM
DISPOSITIF D'OBTURATION D'OUVERTURES DÉFECTUEUSES DANS LE SYSTÈME VASCULAIRE

(30) Priorität: 26.03.2008 DE 102008015781
(43) Veröffentlichungstag der Anmeldung: 15.12.2010
(73) Patentinhaber: Neuss, Malte, 53121 Bonn (DE)
(72) Erfinder: Neuss, Malte, 53121 Bonn (DE)
(74) Vertreter: Thiel, Christian
(86) Internationale Anmeldenummer: PCT/EP2009/002225
(87) Internationale Veröffentlichungsnummer: WO 2009/118182

(56) Entgegenhaltungen:
- EP-A- 1 616 530
- WO-A-01/49213
- WO-A-96/41589
- WO-A-2004/064671
- WO-A-2007/115117
- US-A1- 2002 169 475
- US-A1- 2006 122 647
- US-A1- 2007 129 755

## Beschreibung

Die vorliegende Erfindung betrifft eine Vorrichtung zur interventionellen Therapie von vaskulären Malformationen, beispielsweise zum Verschluss von Septumdefekten im menschlichen oder tierischen Körper. Sie kann bei der Therapie von Herzfehlern mit Links-Rechts-Shunt z. B. eines Atriumseptumdefektes (ASD) oder eines persistierenden Foramen ovale (PFO) mittels Kathetertechniken eingesetzt werden.

Schon seit längerer Zeit ist man bemüht, chirurgisch schwer erreichbare Gefäße mit Hilfe von Kathetertechniken zu verschließen. Ein Kathetereingriff ist für den Patienten weniger belastend als eine Operation und unter Umständen reicht Sedierung aus.

Auch Septumdefekte des Herzens können seit längerem mittels Kathetertechniken verschlossen werden. Die ersten Transkatheter-Verschlüsse von Vorhofseptumdefekten wurden 1976 von King und Mills (Mills N.L., King T.D.; Nonoperative Closure of Left-to-right Shunts; J. Thorac. Cardiovasc. Surg. 72: 371-378, 1976) in Tierversuchen und an Menschen durchgeführt. Die verwendete Verschlussvorrichtung und ein weiteres Schirmsystem, das von Rashkind (Rashkind W.J., Mullins C.E., Hellenbrand W.E., et al; Nonsurgical Closure of Patent Ductus Arteriosus: Clinical Application of the Rashkind PDA Occluder System; Circulation 75: 583-592, 1987) entwickelt wurde, fanden wegen der Größe der Einführschleuse von zunächst 23F bei erst genannter Vorrichtung und der Fixierungshaken bei der Einscheiben-Vorrichtung nach Rashkind und der Doppelschirm nach Rashkind nie breite klinische Anwendung.

Eine Weiterentwicklung dieses Schirmsystems stellt der sechseckige Schirm mit dem Namen Intrasept von der Firm Cardia (www.cardia.com) für den PFO-Verschluss dar. Mit einer doppelten Gelenkverbindung zwischen den beiden Schirmen wird dem unterschiedlich gewinkelten Verlauf des PFO Rechnung getragen. Nachteilig ist für den Verschluss größerer Defekte die fehlende Selbstzentrierung.

Zwei weitere Verschlussvorrichtungen sind das sogenannte "Clamshell Device" nach Lock (Lock J.E., Rome J.J., Davis R. et al; Transcatheter Closure of Atrial Septal Defects, Experimental Studies, Circulation 79: 1091-1099, 1989) und das "Buttoned Devicell nach Sideris (Sideris E.B., Sideris S.E., Fowlkes J.P. et al; Transvenous Atrial Septal Defect Occlusion in Piglets with a "Buttoned" Doubledisk Device; Circulation 81: 312-318, 1990), die im wesentlichen eine schirmartige Form mit sich vom Zentrum aus erstreckenden Metallstreben ohne oder mit Federgelenken als Träger für eine Stoffbespannung aufweisen. Probleme mit dem "Clamshell Device" umfassen spontane Embolisation der Vorrichtung, verbleibende Restshunts und Drahtbrüche. Die Nachteile des "Buttoned Device" sind eine unbequeme Implantationstechnik, verbleibende Restdefekte und Embolisationen.

Aus WO 95/27448 ist eine selbstexpandierbare Vorrichtung auch zum Verschluss von Defektöffnungen oder Gefäßen bekannt, die ebenfalls aus miteinander kreuzenden, oder teilweise geflochtenen Drahtbündeln besteht. Nachteilig an diesem Schirmsystem ist, dass es nicht replazierbar ist und dass durch die sich kreuzenden Drahtfilamente zwangsweise der Einführkatheterdurchmesser vergrößert ist.

Vor Aufgabe der vorliegenden Erfindung US 2006/122647 A1, WO 96/41589 A, WO 2004/064671 A und WO 01/49213 A beschreiben selbstexpandierende Vorrichtungen zum Verschließen von Defektöffnungen im menschlichen oder tierischen Körper, die in einem ersten Zustand die Form eines gestreckten Rohres mit geschlitzten Segmenten und in ein zweiten expandierten Zustand zwei Hohlstrukturen in Form von geöffneten Schirmen aufweisen.

Aus EP 1 616 530 A1 geht eine selbstexpandierende Filtervorrichtung zum Ausfiltern von Thromben aus Blutgefäßen hervor. Der Gefäßfilter hat in der expandierten Form die Struktur eines Hohlkörpers.

WO 2007/115117 A, US 2007/129755 A1 und US 2002/169475 A1 beschreiben Verschlussvorrichtungen für Septumsdefekte.

Aufgabe der vorliegenden Erfindung ist es, eine Vorrichtung zum Verschließen von Defektöffnungen im menschlichen oder tierischen Körper bereitzustellen, welche nicht die Nachteile der oben genannten Vorrichtungen aufweist.

Die Aufgabe wird gelöst durch eine selbstexpandierende Vorrichtung, insbesondere ein Implantat zum Verschließen von Defektöffnungen im menschlichen oder tierischen Körper, die in einem ersten Zustand die Form eines gestreckten Rohr mit geschlitzten Segmenten hat und in einem zweiten Zustand eine verkürzte Form unter Ausbildung wenigstens einer offenen oder (im Wesentlichen) geschlossenen Hohlstruktur mit großer Querausdehnung annimmt, wobei die geschlitzten Segmente des Rohrs einzelne Stege ausbilden, die jeweils mit benachbarten Stegen verbunden sind, so dass im zweiten Zustand eine netzartige Gesamtstruktur entsteht, wobei die Vorrichtung in der Seitenansicht aus mindestens zwei Schirmen mit einem selbstexpandierenden Mittelstück besteht und das Mittelstück im ersten Zustand schräg parallel verlaufende Streben zur Selbstzentrierung aufweist, welche abschnittsweise untereinander verbunden sein können.

Die selbstexpandierende Vorrichtung ist insbesondere ein vaskuläres Implantat. Im ersten Zustand ähnelt die langgestreckte Form mit den geschlitzten Segmenten einem herkömmlichen vaskulären Stent. Im zweiten Zustand, der beispielsweise nach Fortfall eines äußeren Zwangs durch eine Umhüllung oder in einem Katheter angenommen wird, weitet sich dieses gestreckte und geschlitzte Rohr unter erheblicher Verkürzung im zentralen Bereich zu wenigstens einer Hohlstruktur in Form mehrerer Schirme auf, wie sie gebraucht wird, um beispielsweise ein Gefäßdefekt zu verschließen. Bei dieser Umformung behalten das proximale und das distale Ende der Vorrichtung ihren Rohrcharakter im Wesentlichen bei, d. h. nehmen an der Umformung nicht oder nur in geringerem Ausmaß teil.

Im Sinne der Erfindung bezeichnet "distal" den dem behandelnden Arzt oder dem Katheter zugewandten Teil der Vorrichtung, "proximal" den dem Körper zugewandten und vom behandelnden Arzt wegweisenden Teil.

Im aufgeweiteten zweiten Zustand nimmt die erfindungsgemäße Vorrichtung eine stark verkürzte Form unter radialer Aufweitung an. In der Regel ist die Aufweitung so groß, dass der Durchmesser der Vorrichtung ihre Länge im zweiten Zustand übersteigt. Das Ausmaß der Aufweitung hängt allerdings von den Umständen und von der Art und Größe des zu behandelnden Defekts ab.

Bei mit Hilfe eines Lasers vorgeschnittenen Vorrichtung gemäß der Erfindung ist es vorteilhaft, an den Punkten mit großer Richtungsänderung/Spannung schräg parallel verlaufende Streben aus dem Rohr zu schneiden, um die Biegespannung bei der Umformung und beim Aufstellen der Hohlstrukturen zu minimieren. Dies gilt insbesondere für die peripheren Teile der Hohlstrukturen, insbesondere Schirme.

Das gleiche gilt auch für den Mittelteil zwischen zwei schirmartigen, rosettenförmigen Strukturen. Zum einen kann auch hier die Spannung durch schräg parallel verlaufende Streben im Rohr vermindert werden. Zum anderen erlaubt diese Gestaltung aber auch eine Selbstzentrierung der Vorrichtung in einem Defekt und verhindert das Verrutschen des Schirmsystems innerhalb des Defekts. Die schräg parallel verlaufenden Segmente oder Abschnitte im Mittelteil zwischen zwei Doppelschirmen sollten aber abschnittsweise miteinander verbunden sein, um die Stabilität zu erhöhen.

Die erfindungsgemäße Vorrichtung mit zwei schirmartigen rosettenförmigen Strukturen kurzer Länge und großer Querausdehnung ist insbesondere zum Verschluss eines ASD oder PFO mittels Kathetertechniken geeignet. Im Folgenden wird die Erfindung anhand einer solchen doppelten Schirmstruktur beschrieben. Im Mittelteil zwischen diesen beiden schirmartigen Strukturen gibt es eine Einschnürung bestehend aus einer kürzeren oder längeren stentartigen Struktur, die dem Durchmesser und der Form des Defektes angepasst ist.

Diese stentartige Struktur schient den Defekt und verhindert ein seitliches Verrutschen im Defekt. Durch die materialbedingte Klemmkraft zwischen den beiden schirmartigen Strukturen werden jene von beiden Seiten an die restliche Septumwand um den Defekt herum flexibel angedrückt. Dadurch wird sichergestellt, dass die Vorrichtung einfach und schnell implantierbar ist und nicht mehr innerhalb der Öffnung nach der Implantation verschoben werden kann. Die Vorrichtung ist somit wesentlich flexibler einsetzbar und braucht nicht mehr so genau an die Größe und Geometrie der Defektöffnung angepasst werden. Dies ist insbesondere beim Verschluss von intrakardialen Defekten von Bedeutung, da diese Defekte in der Regel keinen kreisförmigen sondern einen ovalen Querschnitt aufweisen.

Vorteilhaft ist es, wenn die stentartige Struktur zwischen den beiden schirmartigen Strukturen sich in diesem weiteren, implantierten Zustand elastisch gegen den Rand der zu verschließenden Öffnung abstützt und dadurch als Selbstzentrierung wirkt.

Zusätzlich kann die Vorrichtung der vorliegenden Erfindung stoffartige Membranen enthalten, die jeweils auf die netzförmige Schirmstruktur aufgebracht sind.

Vorteilhafterweise wird die Vorrichtung durch eine geeignete Mehrfachschlitzung mit drei bis zu 200, vorzugsweise 6 bis 72, Schlitzungen auf der Zirkumferenz mittels Laserschneidverfahren aus einem federelastischem Rohr mit einem Durchmesser von 0,2 bis 5 mm, vorzugsweise 1 bis 3 mm und mit einer Wandstärke von 0,01 bis 0,3 mm, vorzugsweise 0,05 bis 0,15 mm hergestellt, so dass durch die Schlitzung die Anzahl n (3 bis 200) Einzelstege auf der Zirkumferenz entstehen. Die einzelnen Stege sollen zumindest paarweise miteinander verbundenen sein, eine annähernd quadratische oder rechteckige Querschnittsform und eine Länge von 0,5 bis 50 mm, vorzugsweise 1 bis 10 mm aufweisen, und nach der Umformung in den zweiten Zustand insgesamt eine netzförmige Gitterstruktur aufweisen. Je nach äußerer Form können die äußeren Schirmanteile nach Umformung in den zweiten Zustand wie eine Rosette ausgebildet sein. Vorzugsweise wird das Ausgangsrohr an einem oder beiden Enden jeweils auf einer Länge von 0,3 bis 3 mm nicht geschlitzt, um die schirmartige Zweitstruktur einfach wieder zur Passage durch einen Einführkatheter zusammenfalten zu können und zur Aufnahme eines Replaziermechanismus auf der proximalen Seite und zur Ausbildung eines Schirmknopfes auf der anderen distalen Seite. Das Rohr kann dort nur abgerundet und innen hohl zur besseren Führung des Schirmsystems über einen Führungsdraht sein oder mit einem Stopfen zugeschweißt zur Vermeidung scharfer Kanten. Die einzelnen Steglängen können jeweils gleich lang, parallel oder schräg zur Längsachse des Rohres über die gesamte Rohrlänge angeordnet sein oder jeweils leicht versetzt. Die Steglängen können in der Längsrichtung des Rohres aber auch von unterschiedlicher Länge sein.

Für alle Steglängen längs des Rohres im ersten Zustand gilt: Das Produkt aus der maximalen Steglänge multipliziert mit der Anzahl n der Stege soll mindestens dem 3,2-fachen bis dem 9-fachen des jeweiligen Schirmdurchmessers entsprechen, vorzugsweise dem 3,3- bis 5-fachen.

So kann jeweils im Mittelteil zwischen beiden Schirmanteilen sowohl die Anzahl der Stege als auch die Steglänge geringer ausfallen, als in Bereichen mit maximalen Schirmdurchmesser.

Ist eine größere Steifigkeit in bestimmten Schirmabschnitten erwünscht, können dort auch breitere Stege oder Doppelstege vorgesehen werden. Ist eine geringere Steifigkeit einzelner Schirmabschnitte gewünscht, wie im Außenbereich der Einzelschirme, kann dies durch Materialabtragung durch Anbringen zusätzlicher Aussparungen, lokales Abschleifen oder verstärkte Elektropolitur erreicht werden. Die einzelnen Stege können längs ihres Verlaufs eine unterschiedliche Breite aufweisen, gerade sein, leicht gebogen oder s-förmig, sie können zusätzlich mit Kerben oder Ösen versehen sein, um das Annähen des Schirmmaterials zu erleichtern.

Durch eine größere Anzahl von Stegen sowie eine größere Breite der Stege kann die Krafteinwirkung auf den Defektrand zur Entlastung des umgebenden Gewebes möglichst gering gehalten werden. Aufgrund der Selbstzentrierung der Vorrichtung können aber im Gegensatz zu den Vorrichtungen des Standes der Technik auch Defekte mit im Vergleich zum Idealfall zu großen oder zu kleinen Vorrichtungen noch mit hinreichender Sicherheit verschlossen werden.

Wie auch bei Gefäßstents zum Offenhalten von Gefäßen können die Verbindungselemente zwischen den einzelnen Stegen unterschiedlich ausgestaltet werden: X-förmig, H-förmig, C- oder bogenförmig.

Vorzugsweise sollen die einzelnen Stege im zweiten, aufgeweiteten Zustand jeweils insgesamt annähernd viereckige Parallelogramme, Rauten oder Sechsecke ausbilden, die jeweils an der Spitze mit dem nächsten Parallelogramm oder der nächsten Raute verbunden sind, um bei Fehlpositionierung das Zurückziehen des Schirmsystems in den Einführkatheter zu erleichtern, ohne dass sich Schirmteile am Einführkatheter verhaken können. Bei einem Vieleck oder einer gitterförmigen Struktur aus bogenförmigen Streben muss die Gesamtlänge der einzelnen Streben auf beiden Seiten zwischen zwei Verbindungspunkten jeweils gleich sein. Nur so lassen sich die einzelnen Gittersegmente wieder optimal zusammenfalten.

In einer bevorzugten Ausführungsform im zweiten Zustand hat das Schirmsystem in der Seitenansicht eine M-förmige Kontur. Der Stentbereich im Mittelteil zwischen beiden Schirmstrukturen kann auch mit federnden Elementen verbunden sein, um der pulsierenden Wanddickenänderung einer Septumwand besser zu folgen, oder um unterschiedliche Schirmwinkel auszugleichen, ohne dass ein Schirmanteil von der Septumwand absteht, wie es beim Verschluss des peristierenden Foramen ovale (PFO) bei anderen Schirmsystemen häufiger der Fall ist.

Der Stentbereich im Mittelteil zwischen beiden Schirmanteilen kann auch nur aus spiralig angeordneten Stegen bestehen, die sich von selbst unterschiedlichen Defektdurchmessern anpassen, so dass beim Übergang von dem gestreckten Zustand im Implantationskatheter in den expandierten Zustand eine Drehbewegung stattfindet, bei der sich die spiralig angeordneten Stege von der Längsachse radial nach außen bewegen und sich dadurch elastisch an den Rand der zu verschließenden Öffnung abstützen. Durch diese Drehbewegung können sich die beiden Schirmanteile nähern bis hin zur Berührung. Dadurch wird sowohl die zentrierende Abstützung innerhalb der zu verschließenden Öffnung als auch der Formschluss der Vorrichtung an beiden Seiten des die Öffnung umgebenden Gewebes erreicht. Besonders vorteilhaft ist es, wenn die beiden äußeren Schirmstrukturen etwa gleich groß sind, eine runde Form in der Draufsicht aufweisen und in der Seitenansicht jeweils flach und eng an der Defektwand anliegen.

Eine alternative, querovale Schirmform in der Draufsicht auf beiden Seiten ist z. B. zum Verschluss des perimembranösen Ventrikelseptumdefektes von Vorteil, wenn nur wenig Platz zwischen dem Defekt und Klappenstrukturen zur Verfügung steht, die der Schirm keinesfalls berühren soll, da sonst durch die Dauerbelastung die Gefahr einer Klappenperforation besteht. Bei querovalen oder vieleckigen Schirmen ist zur Orientierung der Schirmposition während der Implantation ein röntgendichter Marker am Rand des Schirmes von Vorteil, vorzugsweise in einer definierten Position, etwa am Unterrand.

Die Schirmstruktur wird bevorzugt aus einem Rohr geformt. Das Rohr kann aus jedem federelastischen, medizinisch verträglichen Material sein, aus chirurgischem Stahl und Kobalt-Chrom-Legierungen mit Federeigenschaften oder einem resorbierbaren Metall, wie z. B. Magnesium- oder Eisenlegierungen bestehen oder aus resorbierbarem Kunststoffmaterial, wie z.B. Polygalactid oder Polydioxanon mit Federeigenschaften. Bevorzugt sind jedoch Nickel-Titan-Legierungen mit Formgedächtnis, wie etwa Nitinol. Die Umformung und Federwirkung kann beispielsweise durch eine geeignete Wärmebehandlung erreicht bzw. eingestellt werden. Kunststoffe mit Formgedächtnis sind ausdrücklich einbezogen.

Statt eines Rohres können auch mindestens einmal an den Enden verbundene Drähte oder besser noch Litzen oder Drahtgeflechte verwendet werden, die im ersten Zustand die Form eines Rohrs annehmen. Der Drahtquerschnitt kann beispielsweise rund, oval, halbrund quadratisch oder rechteckig sein und auch über die Länge des Drahtes variieren. Alternativ kann auch ein Kunststoff mit entsprechenden elastischen Eigenschaften verwendet werden, was sich insbesondere bei Elementen, die aus einem Primärrohr geformt sind, anbietet. Die drahtförmigen Elemente können gegebenenfalls mit Platin- oder Gold- oder Wolframdraht umwickelt oder mit Platin- oder Goldringen versehen sein, um den Röntgenkontrast zu erhöhen. Wenn die Schirmelemente aus zusammengefügten Litzen oder Drahtgeflechten gebildet werden, ist es besonders vorteilhaft, in der Litze oder dem Drahtgeflecht Einzeldrähte aus Platin- oder Gold einzuarbeiten zur Erhöhung der Röntgensichtbarkeit. Die einzelnen Drahtelemente der Schirmanteile können seitlich mit den benachbarten Drahtelementen durch Nähte, Umwickeln, Verflechten, durch Hülsen oder Ringe oder Nieten miteinander verbunden sein. Es ist bei zusammengenähten Schirmanteilen vorteilhaft, wenn die einzelnen Schirmstege Ösen oder Bohrungen zur Erleichterung des Zusammennähens enthalten. Auch am Einzelschirm angebrachte Ösen an der Zirkumferenz können das Zusammenfügen mehrer einzelner Schirmanteile ermöglichen. Insbesondere die Verwendung von verdrillten und/oder geflochtenen Mehrfachdrähten oder -litzen vermindert die Bruchanfälligkeit und erhöht die Flexibilität.

Die Schirme oder Schirmanteile aus geflochtenen Drähten können beispielsweise nach allen Methoden der Klöppeltechnik hergestellt werden, aber auch - insbesondere bei einfachen Mustern - maschinell mit Flechtmaschinen.

Zur Erhöhung der Biokompatibilität kann die Schirmstruktur auch beschichtet werden, etwa mit DLC (Diamond like Carbon), mit das Einwachsen fördernden Substanzen oder einer antibiotischen oder thrombotischen Schicht.

Die Vorrichtung kann zumindest einseitig, vorzugsweise jedoch beidseitig mit einer Membran, einem Geflecht, einem Gewirk, einem Flies, einer Folie oder anderen zur Implantation geeigneten Textilien zur Abdichtung eines Schirms oder Doppelschirms versehen sein. Die Membran kann aus Polyester, PTFE, PVDF, Polyurethan, Nylon, oder resorbierbaren Materialien z. B. Polygalactid, Zellulose oder Polydioxanon oder aus feinen Drahtgewirken oder -geflechten aus Nitinol oder anderen Metallen, die resorbierbar oder nicht resorbierbar sind, oder aus Kompositverbindungen aus den oben genannten Materialien bestehen. Die Membran kann entweder eingenäht, eingeflochten, aufgeklebt, mit einer zweiten Membran verklebt oder aufgeschweißt werden.

Bei Verwendung von Drahtlitzen können aufgebrachte Fäden oder Fasern aus Polyester, Nylon, resorbierbaren Nahtmaterialien, Zellulose oder PTFE die Thrombogenität der Schirmstrukturen zum Defektverschluss erhöhen.

Betrachtet man die Draufsicht der Vorrichtung im implantierten Endzustand, so kann die äußere Form der Einzelschirme je nach Anzahl und Anordnung der Stege dreieckig, viereckig (z. B. quadratisch, rechteckig oder rautenförmig) polygonal, sternförmig oder kontinuierlich (z. B. rund, oval oder halbrund) bei einer größeren Anzahl von Stegen auf der Zirkumferenz des Ausgangsrohrs sein.

Zum Verschluss eines Atrium-Septumdefektes (ASD) wird z. B. der Implantationskatheter von der Femoralvene aus durch den rechten Vorhof über den Septumdefekt in den linken Vorhof vorgeführt. Der vordere Schirmanteil wird danach durch Vorschieben der Vorrichtung und Zurückziehen des Implantationskatheters in dem rechten Vorhof langsam rekonfiguriert. Der fast parallel zum Defekt an der Wand des linken Vorhofs stehende vordere Schirmanteil sorgt für einen sicheren Schirmsitz und verhindert ein Durchgleiten dieses Schirmanteils beim Zurückziehen. Der Implantationskatheter ist dabei vorteilhafterweise mit einer derartigen Krümmung zu versehen, dass er senkrecht zur Septumwand steht. Dadurch wird eine Fehlpositionierung des vorderen Schirms auf der falschen Seite des Septums weitgehend vermieden.

Um die Vorrichtung nach einer der oben beschriebenen Ausführungsformen zu implantieren, muss sie zuerst in einen Katheter eingeführt werden, wozu sie gestreckt werden muss. Die Streckung erfolgt durch Auseinanderziehen der Vorrichtung am vorderen und hinteren Ende. Anschließend müssen die Luftblasen durch Spülen aus dem Schirmsystem entfernt werden. Die gestreckte Vorrichtung wird in den Katheter eingeführt. Wenn die Vorrichtung am Zielort aus dem Einführkatheter herausgeschoben wird, rekonfiguriert sie sich wieder im Defekt in ihrem zweiten Zustand.

Die Ausgestaltung der Schirmstrukturen bewirkt, dass sich die Vorrichtung beim Verschluss des Defekts selbst zentriert, was gegenüber den Verschlussvorrichtungen des Standes der Technik einen großen Vorteil darstellt.

An einem Ende der Vorrichtung vorzugsweise an einer Verbindungsstelle kann zur exakteren Positionierung eine Einrichtung zum Replazieren vorgesehen sein, deren Ausgestaltung sich nach der Art des Replaziermechanismus der verwendeten Einführhilfe richtet. Der Replaziermechanismus sollte zentral in der Mitte, und nicht an einer Außenecke oder Kante des Schirms angreifen, damit nach dem Ablösen des Replaziermechanismus dieser Schirm vorzugsweise mit einer Fangschlinge wieder in den Implantationskatheter zurückgezogen werden kann.

Besteht die Vorrichtung im ersten Zustand aus rohrförmig angeordneten Drähten oder Litzen, ist es vorteilhaft diese an beiden Enden jeweils zwischen zwei ringförmigen Röhrchen zu fassen und durch Nähen, Kleben, Löten, Schweißen formschlüssig zu verbinden. Ein weiteres Röhrchen kann dann ein Teil des Replaziermechanismus sein. Die Verwendung von Litze hat den großen Vorteil, dass die Umformkräfte an den Stellen mit großer Biegung im zweiten Zustand wesentlich geringer sind und so die Gefahr eines Draht- oder Stegbruchs wie bei den Ausführungsformen, die aus einem Metallrohr geschnitten werden, wesentlich geringer ist.

Statt einem inneren Rohr, einer Buchse oder einem Ring können an den inneren Streben auch Ösen angebracht sein, die mit einem umlaufenden Faden durch alle Innenösen in einer ringartigen Struktur zusammengehalten werden.

Auch können die einzelnen Schirmanteile flacher aneinander liegen und sind bei Dauerbelastungen durch die Herzkontraktionen besser vor Drahtbrüchen geschützt. Bei Verwendung von einer Drahtlitze hat der Bruch einzelner Drahtfilamente so gut wie keine Auswirkungen auf die Festigkeit der Gesamtstruktur.

Die Einrichtung zum Replazieren kann beispielsweise die Form einer Kugel haben, so in Verbindung mit einer Einführhilfe in Form einer modifizierten Biopsiezange, die diese Kugel umgreift.

Andere mögliche Ausführungsformen der Vorrichtung zum Replazieren sind beispielsweise eine Öse für spezielle Greifwerkzeuge, ein Haken, eine Faden oder Kunststoffverbindung, die durch eine Schneideinrichtung oder Thermolyse mittels Laserenergie getrennt wird, ein Doppelfaden mit Öse, eine Schraubverbindung, eine Klebeverbindung und eine Lötverbindung, die durch Elektrolyse gelöst werden kann oder bevorzugt eine Kupplung, die erst nach Entfernen des inneren Drahtes gelöst werden kann. Dabei ist in dem schirmseitigem Kupplungsteil eine Kerbe eingearbeitet, die das Einfangen des Schirmendes mit einer Fangschlinge erleichtert, damit der Schirm auch nach dem endgültigen Abwurf wieder mittels Kathetertechnik geborgen werden kann, als ein weiterer Sicherheitsaspekt dieses Schirmsystems.

So kann beispielsweise auch ein Schirm in einer Peripherie mit Außenösen ausgestattet sein, durch die ein umlaufender Faden geführt ist, der mit seinen beiden Enden durch einen Einführungskatheter geführt wird und das Zusammenziehen und Zusammenfalten des Schirms erlaubt. Damit wird die erfindungsgemäße Vorrichtung replazierbar und kann vorteilhaft bei Fehlpositionierung wieder in den Einführungskatheter zurückgezogen und ggf. neu platziert werden. Diese Ausführungsform gilt sowohl für geflochtene als auch geschnittene Varianten.

Am gegenüberliegenden Ende des Drahtgerüstes sind die drahtförmigen Elemente bei den entsprechenden Ausführungsformen einfach nur miteinander verbunden, beispielsweise durch eine Kugel, eine Drahtverdrehung mit oder ohne Ösen, durch Löten, Schweißen, Kleben, Umwickeln, Nähen, durch einen Faden, durch eine Buchse, durch Ösen oder einem Ring. Gegebenenfalls kann aber auch eine zweite Replaziereinrichtung vorgesehen sein, wie z. B. eine Öse zur Aufnahme eines Führungsdrahtes für die "Over-the-Wire-Technik". Dabei wird die Vorrichtung über einen Draht geführt und kann so sicherer positioniert und auch nach Dekonnektion einfach wieder - auch interventionell - entfernt werden. Erst nach vollständig korrektem Sitz des Doppelschirms wird zuletzt diese Drahtführungsschiene wieder entfernt. Die drahtförmigen Elemente müssen nicht direkt miteinander verbunden sein, es kann auch eine indirekte Verbindung z. B. über die Schirmbespannung bestehen oder durch eine Nahtverbindung. Dies hat den Vorteil, dass das Rekonfigurieren des zweiten Schirmanteils auf einer Seite des Septums langsam erfolgt, um ein schlagartiges Entspannen der Vorrichtung zu vermeiden.

Bei weiteren Ausführungsformen der erfindungsgemäßen Vorrichtung besteht die Vorrichtung aus zwei ungleich großen Schirmanteilen mit breiterem Mittelstück in der Seitenansicht, aus einer Kombination von einem Schirmanteil und einem Pfropfen, oder aus einer kugelförmigen Außenform mit oder ohne eine Bespannung, so dass funktionell ein Verschlußpfropfen zum Beispiel für das linke Herzohr entsteht.

Zum Verschluss von Ventrikelseptumdefekten sind Schirmformen mit einem annähernd zylindrischem Mittelteil mit dem Durchmesser des Defekts und zwei Außenschirmen vorteilhaft. Die äußere Schirmform kann dann auch oval ausgebildet werden, um angrenzende Klappenstrukturen zu schonen. Ein besonderer röntgendichter Markierungsring am Oberrand oder Unterrand des Schirms sollte dann die Lagekontrolle des Verschlußsystems bei der Implantation erleichtern. Je nach zu verschließendem Defekt kann das zylindrische Mittelstück auch schräg ausgebildet werden, so dass ein insgesamt ein asymmetrischer Schirm entsteht.

An den Außenrändern des proximalen und/oder des distalen Schirms können sich auch Ösen befinden, mit denen einzelne Schirmanteile mit einem Faden oder Draht mit einer Nahtverbindung zusammengefügt werden können. Bei dieser Ausführungsform, bei der einzelne flache Schirmanteile mit Nähten zusammengefügt werden, ist es auch vorteilhaft, die einzelnen flachen Schirmanteile aus einem Blech auszuschneiden oder zu stanzen, aus einer Kunststoffplatte zu fertigen oder die flachen Schirmanteile aus Kunststoff zu spritzen.

In einer weiteren Ausführungsform kann die erfindungsgemäße Vorrichtung auf beiden Seiten mit einem Replaziermechanismus versehen sein. Dies ist besonders für die Implantation ungleich großer Schirme vorteilhaft, da so jeder Schirm sowohl von venöser als auch von arterieller Seite aus entsprechend der anatomischen Gegebenheiten sicher implantiert werden kann. Auch können dann im Rahmen einer Loop-Procedure von beiden Seiten des Defektes die Schirmanteile jeweils langsam an der Defektwand rekonfiguriert werden.

In einer weiteren Ausführungsform der erfindungsgemäßen Vorrichtung können eine oder beide Schirme jeweils ein Loch mit definiertem Durchmesser zwischen den Streben und der Schirmmembran aufweisen, für den Fall, dass der Defekt nicht vollständig verschlossen werden soll und ein definierter Restfluss über den Defekt gewünscht wird. Ein Pfropfen mit einem definierten Lumen kann auch zur Strömungsreduktion bei Gefäßen eingesetzt werden. Gegebenenfalls kann in dieses Lumen auch eine Klappenstruktur integriert werden, um eine Rückströmung zu verhindern.

Bei diesen alternativen Ausführungsformen können dieselben Materialien wie bei den anderen Ausführungsformen verwendet werden. Lediglich im zweiten Zustand ist die seitliche Form unterschiedlich ausgebildet.

Das Schirmgerüst der erfindungsgemäßen Vorrichtung kann mit einem Gewebe, einem Geflecht oder einer Folie in Form eines Doppelschirms komplett überzogen sein, oder es kann auch nur einer der Schirmanteile innen und/oder außen bespannt sein. Außerdem können Teile des Schirms mit thrombogenen Fäden versehen sein oder das komplette Schirmgerüst in Schaumstoff eingebettet sein. Als Materialien kommen beispielsweise in Frage:
PETP (Polyethylenglykolterephthalat, z. B. Dacron TM), Polyamid (z. B. Nylon), PTFE (Polytetrafluorethylen, z. B. Teflon TM), Seide, deoxigenierte Zellulose, resorbierbarer Kunststoff (z. B. Polygalaktid, Polydioxanon (z. B. PDS TM) als Gewebe, mikroporöse Polyurethanfolie, flexibles Drahtgeflecht aus dünnstem Stahl oder Nitinoldraht oder Kombinationen aus genannten Materialien. Für die Schaumstoffeinbettung eignet sich beispielsweise Polyurethanschaumstoff und Polyvinylschaumstoff, (z. B. Ivalon TM). Die Bespannung kann am Schirmgerüst über Ösen, die an den Streben oder Verbindungspunkten angebracht sind, durch Nähen, Umwickeln, Pressen, Kleben, Schweißen, Löten, Aufschrumpfen und Tauchen befestigt werden. Wenn das Schirmgerüst aus einer locker geflochtenen Drahtlitze gefertigt ist, kann die Bespannung einfach durch Umnähen der durch Einzeldrähte gebildeten Schlaufen der Litze befestigt werden. Bei geflochtenen Streben ist ein Zusammenflechten mit Nachbarstreben in Teilbereichen vorteilhaft. Dafür können auch eingeflochtene Ösen vorgesehen werden.

Die Gesamtdicke der Drahtlitze beträgt 0,01-0,06 mm, vorzugsweise 0,02-0,035 mm. Der minimale Implantationskatheterdurchmesser beträgt 3-9F (1-3 mm).

Die erfindungsgemäße Vorrichtung kann beispielsweise zum Verschluss eines Vorhofseptumdefekts (ASD), eines Ventrikelseptumdefekts (VSD), eines persistierenden Ductus ateriosus Botalli (PDA) oder einer arteriovenösen Malformation verwendet werden, wobei die Ausführungsform aus an beiden Enden verbundenen draht- oder litzenförmigen Elementen sich insbesondere zum Verschluss von großen PDA, VSD und arteriovenösen Malformationen eignet.

Die Erfindung soll nun anhand einiger Abbildungen veranschaulicht werden. Es versteht sich, dass die einzelnen Elemente und Ausführungsformen jeweils auch anders miteinander kombiniert werden können, wann immer möglich und sinnvoll.

Die Darstellungen von Figuren 1 bis 6 entsprechen in ihrem Mittelstück nicht den Vorgaben der Erfindung, dienen aber der Erläuterung des zur Anwendung kommenden Prinzips.
- Figur 1: zeigt eine Seitenansicht eine rohrförmige Ausführungsform der Erfindung mit einem Implantat mit jeweils gleichlangen Stegen im gestreckten Zustand,
- Figur 1a: zeigt eine Vergrößerung des geschlitzten Rohres im Ausschnitt,
- Figuren 2 und 3: zeigen, wie sich das Implantat einer Ausführungsform gemäß Figur 1 als Doppelschirm beim Herausschieben aus einem Katheter und beim anschließenden Dekonnektieren verhält,

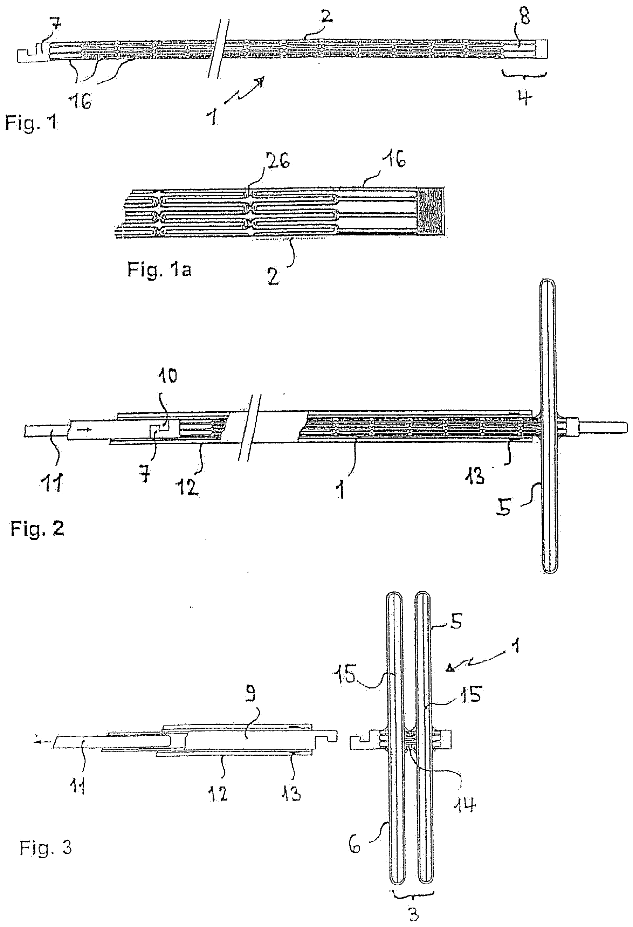

- Figur 2: zeigt eine Seitenansicht der Ausführungsform gemäß Figur 1 in einem Katheter mit einem teilweise rekonfiguriertem Doppelschirm,
- Figur 3: zeigt eine Seitenansicht der Ausführungsform gemäß Figur 1, mit einem dekonnektierten, komplett rekonfigurierten Doppelschirm mit jeweils gleich großen Durchmessern des proximalen und distalen Schirms mit einem schmalen Mittelstück,

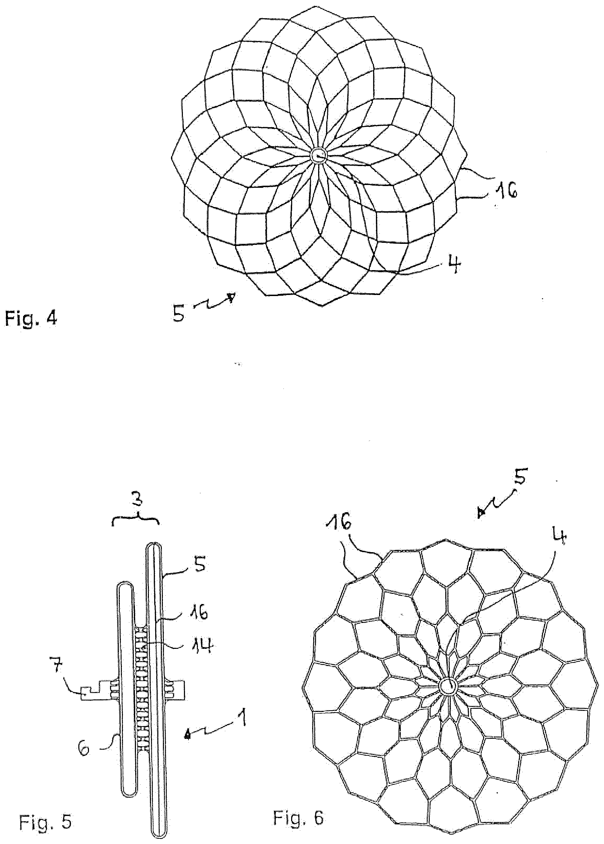

- Figur 4: zeigt eine Ansicht des vorderen Schirmanteils von vorne mit gleich langen Stegen gemäß Figur 3 im rekonfigurierten Zustand,
- Figur 5: zeigt eine Ansicht einer weiteren Ausführungsform des komplett rekonfigurierten Doppelschirms gemäß Figur 1 mit breitem Mittelstück und einem großen proximalen Schirm und einem kleineren distalen Schirm,
- Figur 6: zeigt eine Ansicht des vorderen Schirmanteils von vorne gemäß Figur 5 im rekonfigurierten Zustand mit teilweise unterschiedlich langen Stegen entsprechend des jeweiligen Schirmdurchmessers,

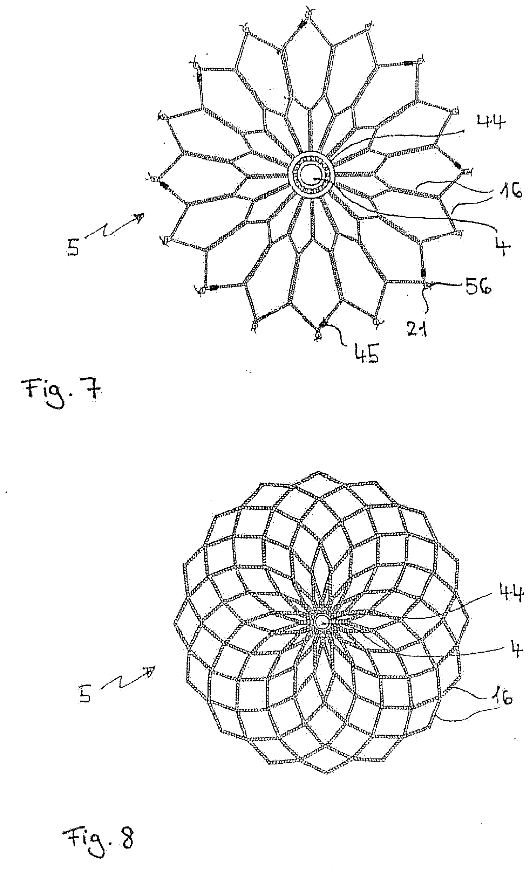

- Figur 7: zeigt eine Ansicht des vorderen Schirmanteils von vorne gemäß Figur 1 im rekonfigurierten Zustand mit aus mehreren Einzeldrähten geflochtenen oder gewickelten Stegen unterschiedlicher Länge,
- Figur 8: zeigt eine Ansicht des vorderen Schirmanteils von vorne gemäß Figur 1 im rekonfigurierten Zustand mit gewickelten Stegen gleicher Länge.

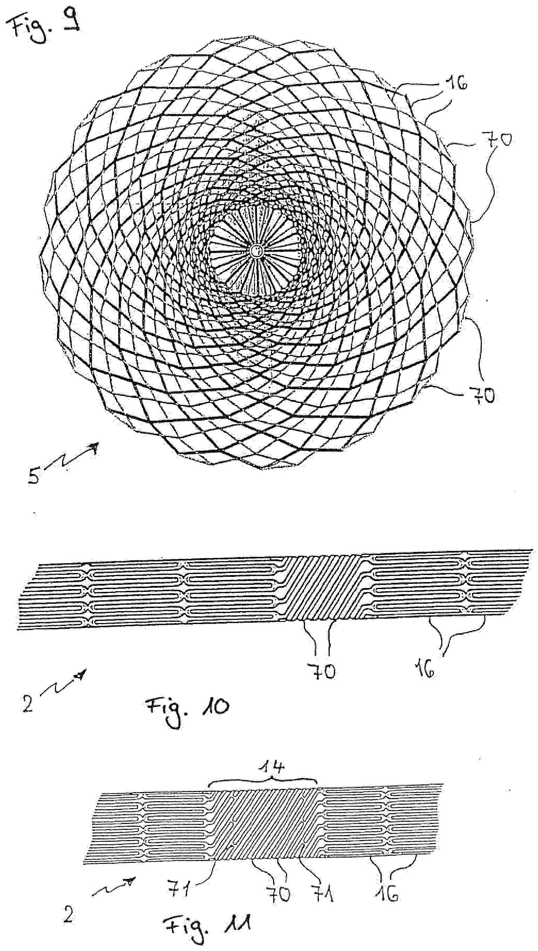

- Figur 9: zeigt einen aus einem Rohr geschnittenen Schirm in der Draufsicht mit in der Peripherie schräg verlaufenden Stegen zur Spannungsminderung,
- Figur 10: einen geschnittenen Rohrabschnitt im ersten Zustand mit schräg parallel verlaufenden Stegen zur Spannungsminderung,
- Figur 11: einen Rohrabschnitt mit schräg parallel verlaufenden Stegen im Mittelteil zur Selbstzentrierung, und

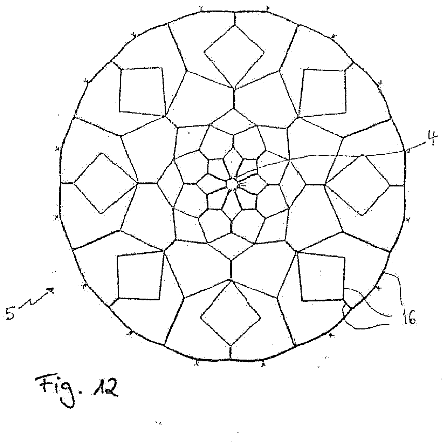

- Figur 12: einen aus Metalllitze geflochtenen Schirm in der Draufsicht, bei dem die zentralen Rauten über Ösen und einen Faden miteinander verbunden sind.

Nachfolgend werden die einzelnen Abbildungen noch detailliert erläutert:
Fig. 1 zeigt in der Seitenansicht eine Ausführungsform einer selbstexpandierenden Vorrichtung in Form eines geschlitzten Rohres 2 in einem ersten gestreckten Zustand.

Bei dieser Ausführungsform des Implantats 1 sind alle Stege 16 jeweils gleich lang. Beim proximalen Endstück 4 befinden sich breitere Schlitze 8 und weniger breite Stege 16 auf der Zirkumferenz, um die Steifigkeit des Implantats 1 in Bereichen mit großer Biegung in einem zweiten Zustand nach Wärmeumformung zu verringern. Am distalen Ende befindet sich eine Implantatkupplung 7.

Fig. 1a zeigt eine Vergrößerung eines Ausschnitts des wie bei Stents geschlitzten Rohres 2 mit gleich langen Stegen 16, die in dieser Ausführungsform seitlich jeweils abwechselnd mit X-förmigen Verbindungselementen 26 miteinander verbunden sind. Parallel zueinander verlaufende Stege 16 bilden jeweils ein (Rohr)Segment.

Fig. 2 zeigt in einer Seitenansicht ein Implantat 1 mit einem komplett rekonfigurierten proximalen Schirm 5 außerhalb des Einführkatheters 12. Das im Einführkatheter 12 gestreckte Implantat 1 ist am distalen Ende mit seiner Implantatkupplung 7 in die Pusherkupplung 10 eingehakt und mit dem Pusher 9 durch einen inneren Führungsdraht 11 fixiert. Der Einführkatheter 12 weist am proximalen Ende einen Markerring 13 auf.

Fig. 3 zeigt in einer Seitenansicht ein dekonnektiertes Implantat 1 gemäß Fig. 1 in einer Ausführungsform als Doppelschirm 3, bei dem der proximale Schirm 5 und der distale Schirm 6 jeweils einen jeweils gleich großen Durchmesser haben. Zwischen ihnen befindet sich ein schmales Mittelstück 14. Im proximalen Schirm 5 und distalen Schirm 6 befindet sich jeweils eine Membran 15 zum besseren Verschluss von Defekten. Die Pusherkupplung 10 befindet sich außerhalb des Einführkatheters 12. Durch Zurückziehen des Führungsdrahtes 11 in dem Pusher 9 wurde das Implantat 1 mit der Implantatkupplung 10 vom Pusher 9 dekonnektiert. Am Ende des Einführkatheters 12 befindet sich ein Markerring 13.

Fig. 4 zeigt eine Ansicht der des Implantats 1 gemäß Fig. 3 in Form eines Doppelschirms von vorne, der in der dargestellten Ausführungsform mit einer Wärmebehandlung aus einem 32-fach geschlitzten Rohr hergestellt wird. Der vordere und hintere Anteil des proximalen und distalen Schirms wird in der Zirkumferenz jeweils aus 32 Stegen 16 gebildet. Die Schirmbreite bei dieser Ausführung besteht aus jeweils 7 Steglängen. Deshalb beträgt die erforderliche Gesamtlänge für das geschlitzte Rohr 2 für dieses Implantat 1 in Form eines Doppelschirms 3 vier Mal die 7-fachen Länge eines Steges, entsprechend 28 Mal der Länge eines einzelnen Steges + der Länge für das proximale Endstück 4 + der Länge für das distale Ende mit der Implantatkupplung 7 + das Mittelstück 14.

Bei dieser Ausführungsform mit jeweils gleich langen Stegen 16 bilden die bei den Schirmanteilen verwendeten Stege untereinander jeweils viereckige Rauten. In Bereichen mit großen Umformkräften beim proximalen Endstück 4 und der Implantatkupplung 7 kann zur Erhöhung der Flexibilität die Breite der einzelnen Stege oder die Steifigkeit durch Längslöcher verringert werden, die Wandstärke durch Elektropolitur verdünnt oder die Anzahl der Stege wird reduziert. Auch am Außenrand des proximalen und distalen Schirms ist es sinnvoll zur Erhöhung der Flexibilität und Federeigenschaften des Doppelschirms die einzelnen Verbindungselemente durch konstruktive Maßnahmen in der Breite oder Wandstärke zu reduzieren.

Fig. 5 zeigt in einer Seitenansicht das Implantat 1 gemäß Fig. 1 in einer weiteren Ausführungsform als Doppelschirm 3. mit ungleichem Durchmesser des proximalen und distalen Schirms. In dieser Ausführungsform hat der proximale Schirm 5 einen größeren Durchmesser als der distale Schirm 6. Zwischen ihnen befindet sich ein breites Mittelstück 14. Im proximalen Schirm 5 befindet sich eine Membran 15 zum besseren Verschluss von Defekten.

Fig. 6 zeigt eine Ansicht des Doppelschirms gemäß Fig. 5 von vorne. In dieser Ausführungsform wird er ebenfalls aus einem 32-fach geschlitzten Rohr hergestellt. Der vordere und hintere Anteil des proximalen und distalen Schirms wird in der Zirkumferenz jeweils aus 32 Stegen gebildet und die Schirmbreite in dieser Ausführung besteht aus jeweils 8 Steglängen.

Bei dieser Ausführungsform mit jeweils unterschiedlich langen Stegen 16, je nach Abstand zum Schirmzentrum mit dem proximalen Endstück 4 bilden die bei den Schirmanteilen verwendete Stege 16 untereinander jeweils Sechsecke.

Fig. 7 zeigt eine Ansicht einer Ausführungsform eines Implantats 1 in Form eines Doppelschirms 3 mit Abbildung der Vorderseite eines proximalen Schirms 5 von proximal. Bei dieser erfindungsgemäßen Variante besteht die gesamte Implantat aus Stegen unterschiedlicher Länge, die aus mehreren Einzeldrähten geflochtenen oder gewickelt wurden. In der Mitte des proximalen Schirms 5 werden die geflochtenen oder gewickelten Stege 16 mit einer Buchse 44 zum proximalen Endstück 4 zusammengefasst. Die Schirmbreite wird in dieser Ausführungsform aus einer Gesamtlänge von jeweils vier Stegen 16 gebildet. Hierbei sind am Außenrand des proximalen Schirms acht röntgenopake Marker angebracht, um die Schirmpositionierung unter Röntgenkontrolle zu vereinfachen. An den Außenrändern des proximalen und distalen Schirms können sich auch Ösen 21 befinden, mit denen einzelne Schirmanteile mit einem Faden oder Draht mit einer Nahtverbindung 56 zusammengefügt werden können. Bei einer Ausführungsform, bei der einzelne flache Schirmanteile mit Nähten zusammengefügt werden, ist es auch vorteilhaft, die einzelnen flachen Schirmanteile aus einem Blech auszuschneiden oder zu stanzen aus einer Kunststoffplatte zu fertigen oder die flachen Schirmanteile aus Kunststoff zu spritzen.

Fig. 8 zeigt eine Ansicht einer Ausführungsform eines Implantats 1 in Form eines Doppelschirms 3 wie Fig 4 mit Abbildung der Vorderseite eines proximalen Schirms 5 von vorne. Jedoch besteht bei dieser erfindungsgemäßen Variante das gesamte Implantat aus Stegen 16 gleicher Länge, die entweder aus mehreren Einzeldrähten geflochtenen oder gewickelt wurden. In der Mitte des proximalen Schirms 5 werden die geflochtenen oder gewickelten Stege 16 mit einer Buchse 44 zum proximalen Endstück 4 zusammengefasst. Die Schirmbreite wird in dieser Ausführungsform aus einer Gesamtlänge von jeweils sieben Stegen 16 gebildet.

Fig. 9 zeigt ein Implantat gemäß Fig. 3 in Form eines Doppelschirms in der Draufsicht, das aus einem mehrfach geschlitzten Rohr hergestellt wird. Der Schirm wird von einer Vielzahl von jeweils gleich langen Stegen 16 gebildet, die zu einer Vielzahl von Rauten zusammengeführt sind. Im peripheren Bereich werden die distale und die proximale Seite des Schirms über schräg gestellte Stege 70 miteinander verbunden, die dazu führen, dass die proximale und die distale Seite des Schirms um die Länge dieser schräg gestellten Stege 70 gegeneinander versetzt sind. Diese Anordnung führt zu einer deutlichen Verminderung der Spannung im peripheren Bereich der Schirme und damit zu einer Verminderung der Bruchgefahr.

Fig. 10 zeigt das geschnittene Rohr für die Ausführungsform gemäß Fig. 45 im ersten, nicht expandierten Zustand. Das Rohr 2 weist die Vielzahl von gleich langen Stegen 16 auf, die zu einzelnen Segmenten des Rohres geordnet sind. Eines dieser Segmente weist dann schräg parallel verlaufende Stege 70 auf, die bei der Expansion eine Verdrillung der beiden Schirmhälften gegeneinander und damit eine Spannungsminderung bewirken.

Fig. 11 zeigt eine weitere Ausführungsform in Form eines geschnittenen bzw. geschlitzten Rohres 2 mit schräg parallel verlaufenden Stegen 70 im Mittelteil 14 einer erfindungsgemäßen Vorrichtung 1. Die einzelnen parallel verlaufenen Stege 70 sind abschnittsweise über Brücken 71 miteinander verbunden, um eine gewisse Stabilität zu erzielen. Das Mittelteil kommt zwischen zwei Schirme 5 und 6 einer erfindungsgemäßen Vorrichtung zum Liegen und passt sich bei schräg gestellten Stegen 70 relativ leicht an die Dimension eines zu verschließenden Durchgangs an. Es erfolgt eine Selbstzentrierung des Implantats.

Fig. 12 zeigt einen geflochtenen Schirm 5 in der Draufsicht mit einer Vielzahl von Stegen 16. Die Stege sind im gezeigten Fall unterschiedlich lang. Im Zentrum sind die einzelnen Stege über endständig angeordnete Ösen und eine durch diese Öse geführten Faden zu einer ringförmigen Struktur zusammengeführt, was die Struktur fixiert und im ersten Zustand die Einnahme der Rohrform 2 erlaubt. Die Flächenstruktur der einzelnen Stege 16 erlaubt eine große Flexibilität und vermeidet Brüche aufgrund zu hoher Biegebelastung wie auch Ermüdungsbrüche.

Es versteht sich, dass die in den Abbildungen angesprochenen einzelnen Ausführungsformen und Merkmale Beispiele sind. Die Erfindung umfasst darüber hinaus jede sinnvolle Kombination von Merkmalen aus der Beschreibung, die unter die Randbedingung des Anspruchs 1 fällt.

## Patentansprüche

1. Selbstexpandierende Vorrichtung, insbesondere Implantat zum Verschließen von Defektöffnungen im menschlichen oder tierischen Körper, die in einem ersten Zustand die Form eines gestreckten Rohres (2) mit geschlitzten Segmenten hat und in einem zweiten Zustand eine verkürzte annimmt, die wenigstens eine offene oder im Wesentlichen geschlossene Hohlstruktur (3) mit großer Querausdehnung definiert, wobei die geschlitzten Segmente des Rohres (2) einzelne Stege (16) ausbilden, die jeweils mit benachbarten Stegen (16) verbunden sind, so dass im zweiten Zustand eine netzartige Gesamtstruktur entsteht, **dadurch gekennzeichnet, dass** die Vorrichtung in der Seitenansicht aus mindestens zwei Schirmen (5, 6) mit einem selbstexpandierenden Mittelstück (14) besteht und das Mittelstück (14) im ersten Zustand schräg parallel verlaufende Streben zur Selbstzentrierung aufweist, welche abschnittsweise untereinander verbunden sein können.

2. Vorrichtung nach Anspruch 1, **gekennzeichnet durch** im ersten Zustand segmentweise schräg parallel verlaufende Streben zur Spannungsreduktion und/oder Selbstzentrierung.

3. Vorrichtung nach Anspruch 2, **gekennzeichnet durch** zwei Hohlstrukturen (3) in Form eines proximalen (5) und eines distalen Schirms (6).

4. Vorrichtung nach einem der Ansprüche 1 bis 3, **dadurch gekennzeichnet, dass** sie aus verbundenen Stegen (16) von Einzeldrähten, Mehrfachdrähten oder Drahtlitzen gebildet wird, die an einem oder beiden Enden ring- oder rohrförmig zusammengefasst sind.

5. Vorrichtung nach Anspruch 4, **dadurch gekennzeichnet, dass** die Stege (16) aus verdrillten und/oder geflochtenen Mehrfachdrähten oder Mehrfachlitzen bestehen.

6. Vorrichtung nach Anspruch 5 in Form eines Geflechts oder Gewebes.

7. Vorrichtung nach einem der vorstehenden Ansprüche, **dadurch gekennzeichnet, dass** an den Stegen (16) Ösen (24) angeordnet sind und/oder Stege (16) über Ösen (24) verbunden sind.

8. Vorrichtung nach einem der Ansprüche 1 bis 3, **dadurch gekennzeichnet, dass** die Vorrichtung (1) mit den verbundenen Stegen (16) aus einem Rohr durch Laserschneiden gebildet ist.

9. Vorrichtung nach einem der vorstehenden Ansprüche, **dadurch gekennzeichnet, dass** sie im zweiten Zustand gegen den Rand der zu verschließenden Öffnung in radialer Richtung deformierbar ausgebildet ist, so dass sie sich gegen den Rand abstützen kann und annähernd zentrisch innerhalb der Öffnung positionierbar ist.

10. Vorrichtung nach einem der vorstehenden Ansprüche, **dadurch gekennzeichnet, dass** sie an mindestens einem Ende eine Kupplung (7) aufweist.

11. Vorrichtung nach Anspruch 10, **dadurch gekennzeichnet, dass** die Kupplung (7) durch einen innen liegenden Führungsdraht (11) gesichert ist.

12. Vorrichtung nach einem der vorstehenden Ansprüche, **dadurch gekennzeichnet, dass** sie im zweiten Zustand eine rautenförmige oder sechseckige Gitterstruktur aus einzelnen Streben mit Verbindungselementen aufweist.

13. Vorrichtung nach einem der vorstehenden Ansprüche, **dadurch gekennzeichnet, dass** Teile der Hohlstruktur (3, 5, 6) aus einem Blech oder Folie geschnitten werden und die einzelnen Teile zur Hohlstruktur (6) zusammengenäht werden.

14. Vorrichtung nach einem der vorstehenden Ansprüche, **dadurch gekennzeichnet, dass** sie ganz oder teilweise aus Formgedächtnismetallen, insbesondere Nitinol, besteht.

15. Vorrichtung nach einem der vorstehenden Ansprüche, **dadurch gekennzeichnet, dass** sie in Teilbereichen mit einer Membran bespannt ist.

16. Vorrichtung nach einem der vorstehenden Ansprüche, **dadurch gekennzeichnet, dass** sie replazierbar/explantierbar ausgebildet ist.

17. Vorrichtung nach Anspruch 16, **dadurch gekennzeichnet, dass** die Hohlstruktur (3, 5, 6) an ihren peripheren Streben Ösen aufweist, durch die ein umlaufender Faden geführt ist.

## Claims

1. A self-expanding device, in particular implant, for closing of defect openings in human or animal bodies that, in a first state, has the shape of an elongated tube (2) with slit segments and, in a second state, takes on a shorter shape, that defines at least one open or essentially closed hollow structure (3) with a larger lateral extension, whereby the slit segments of tube (2) form individual strips (16) that are respectively connected to adjacent strips (16) so that an overall net-like structure emerges in the second state, **characterized in that** the device consists of at least two umbrellas (5, 6) in a side view with a self-expanding middle piece (14) and that the middle piece (14) in the first state exhibits strips that run diagonally parallel for self-centring which can be connected, section-wise, amongst themselves.

2. Device according to Claim 1, **characterized by** strips that run diagonally parallel, segment-wise, in the first state, in order to reduce tension and/or for self-centring.

3. Device according to Claim 2, **characterized by** two hollow structures (3) in the form of a proximal (5) and distal (6) umbrella.

4. Device according to one of Claims 1 to 3, **characterized in that**, they are formed from connected strips (16) of individual wires, multiple wires or flexible wires that are merged into an annular or tubular shape at both ends.

5. Device according to Claim 4, **characterized in that**, the strips (16) consist of twisted and/or braided multiple wires or multiple slits.

6. Device according to Claim 5 in the form of a netting or fabric.

7. Device according to one of the previous Claims, **characterized in that**, loops (24) are located at the strips (16) and/or strips (16) are connected by loops (24).

8. Device according to one of Claims 1 to 3, **characterized in that**, the device (1) is formed by connected strips (16) from a tube using laser beam cutting.

9. Device according to one of the previous Claims, **characterized in that**, in the second state, they are designed to deform in radial direction against the wall of the opening to be closed so that they can support themselves against the wall and can be positioned approximately centrally within the opening.

10. Device according to one of the above Claims, **characterized in that**, it exhibits a coupler (7) at at least one end.

11. Device according to Claim 10, **characterized in that**, the coupler (7) is secured by an inner guidewire (11).

12. Device according to one of the above Claims, **characterized in that**, it exhibits a rhombus-shaped or hexagonal lattice structure from individual strips with connecting elements in the second state.

13. Device according to one of the above Claims, **characterized in that**, parts of the hollow structure (3, 5, 6) are cut from a sheet or foil and the individual parts are stitched together into a hollow structure (6).

14. Device according to one of the above Claims, **characterized in that**, it consists entirely or partially of shape memory materials, particularly nitinol.

15. Device according to one of the above Claims, **characterized in that**, it is covered in sub-areas by a membrane.

16. Device according to one of the above Claims, **characterized in that**, it is designed in such a manner that it can be repositioned/explanted.

17. Device according to Claim 16, **characterized in that**, the hollow structure (3, 5, 6) has loops at its peripheral strips through which a circumferential thread is guided.

## Revendications

1. Dispositif auto-expansible, notamment implant destiné à fermer des ouvertures pathologiques dans des corps humains ou animaux, qui possède dans un premier état la forme d'un tube étiré (2) doté de segments fendus et, dans un deuxième état, en adopte une plus courte qui définit au moins une structure creuse (3) ouverte ou sensiblement fermée ayant une grande extension transversale, les segments fendus du tube (2) forment des éléments jointifs (16) individuels qui sont respectivement reliés à des éléments jointifs (16) voisins, de sorte qu'une structure globale de type filet se produit dans le deuxième état, **caractérisé en ce que** le dispositif, vu de côté, se compose d'au moins deux écrans (5, 6) avec une pièce centrale (14) auto-expansible et la pièce centrale (14), dans le premier état, possède des entretoises qui s'étendent parallèlement en biais pour l'auto-centrage, lesquels peuvent être reliés entre eux dans certaines portions.

2. Dispositif selon la revendication 1, **caractérisé par** des entretoises qui s'étendent parallèlement en biais par segments dans le premier état pour la réduction des contraintes et/ou l'auto-centrage.

3. Dispositif selon la revendication 2, **caractérisé par** deux structures creuses (3) sous la forme d'un écran proximal (5) et d'un écran distal (6).

4. Dispositif selon l'une des revendications 1 à 3, **caractérisé en ce qu'**il est formé à partir d'éléments jointifs (16) reliés de fils individuels, de fils multiples ou de torons de fils qui sont assemblés en forme d'anneau ou de tube au niveau d'une ou des deux extrémités.

5. Dispositif selon la revendication 4, **caractérisé en ce que** les éléments jointifs (16) se composent de fils multiples ou de torons multiples torsadés et/ou tressés.

6. Dispositif selon la revendication 5 sous la forme d'un treillis ou d'un tissage.

7. Dispositif selon l'une des revendications précédentes, **caractérisé en ce que** des oeillets (24) sont disposés au niveau des éléments jointifs (16) et/ou des éléments jointifs (16) sont reliés par des oeillets (24).

8. Dispositif selon l'une des revendications 1 à 3, **caractérisé en ce que** le dispositif (1) avec les éléments jointifs (16) reliés est formé à partir d'un tube par découpage au laser.

9. Dispositif selon l'une des revendications précédentes, **caractérisé en ce que** dans le deuxième état, il est réalisé de manière déformable dans la direction radiale contre le bord de l'ouverture à fermer, de sorte qu'il peut prendre appui contre le bord et est positionnable de manière approximativement centrée à l'intérieur de l'ouverture.

10. Dispositif selon l'une des revendications précédentes, **caractérisé en ce qu'**il possède un coupleur (7) à au moins une extrémité.

11. Dispositif selon la revendication 10, **caractérisé en ce que** le coupleur (7) est bloqué par un fil de guidage (11) se trouvant à l'intérieur.

12. Dispositif selon l'une des revendications précédentes, **caractérisé en ce que** dans le deuxième état, il possède une structure en treillis en forme de losange ou hexagonale composée d'entretoises individuelles avec des éléments de liaison.

13. Dispositif selon l'une des revendications précédentes, **caractérisé en ce que** des parties de la structure creuse (3, 5, 6) sont découpées dans une tôle ou un film et les parties individuelles sont cousues ensemble pour former la structure creuse (6).

14. Dispositif selon l'une des revendications précédentes, **caractérisé en ce qu'**il se compose en totalité ou en partie de métaux à mémoire de forme, notamment du nickel-titane.

15. Dispositif selon l'une des revendications précédentes, **caractérisé en ce qu'**une membrane est tendue sur lui dans des zones partielles.

16. Dispositif selon l'une des revendications précédentes, **caractérisé en ce qu'**il est configuré pour pouvoir être repositionné/explanté.

17. Dispositif selon la revendication 16, **caractérisé en ce que** la structure creuse (3, 5, 6) possède au niveau de ses entretoises périphériques des oeillets à travers lesquels est passé un fil circonférentiel.
